# EUROPEAN PATENT SPECIFICATION

(11) **EP 2 968 939 B1**
(45) Date of publication and mention of the grant of the patent: **26.02.2020**
(21) Application number: 14763828.2
(22) Date of filing: 17.03.2014
(51) Int. Cl.: A61N 1/36, A61N 2/00, A61N 1/05

(54) **SPINAL NERVE STIMULATION RINGS FOR REHABILITATION OF PATIENTS WITH SPINAL TRAUMA AND STROKE**
SPINALNERVEN-STIMULATIONSRINGE ZUR REHABILITATION VON PATIENTEN MIT RÜCKENMARKSTRAUMA UND SCHLAGANFALL
ANNEAUX DE STIMULATION DES NERFS RACHIDIENS POUR LA RÉÉDUCATION DE PATIENTS SOUFFRANT D'UN TRAUMATISME DE LA MOELLE ÉPINIÈRE ET AYANT SUBI UN ACCIDENT VASCULAIRE CÉRÉBRAL

(30) Priority: 15.03.2013 US 201313838180
(43) Date of publication of application: 20.01.2016
(73) Proprietor: University Of Rochester, Rochester, NY 14642 (US)
(72) Inventor: HUANG, Jason, Haitao, Pittsford, NY 14534 (US); DAYAWANSA, Samantha, Temple, TX 76502 (US)
(74) Representative: Michalski Hüttermann & Partner Patentanwälte mbB
(86) International application number: PCT/US2014/030462
(87) International publication number: WO 2014/145659

(56) References cited:
- WO-A1-2012/064968
- WO-A1-2012/172545
- WO-A2-2008/088985
- US-A- 5 030 225
- US-A1- 2008 046 055
- US-A1- 2008 046 055
- US-A1- 2010 057 178
- US-A1- 2010 324 642
- US-A1- 2012 330 391
- US-B1- 6 169 924

## Description

### TECHNICAL FIELD

The present invention relates generally to medical devices, and more particularly to a medical device for rehabilitation of patients with nerve related disorders.

### BACKGROUND ART

Spinal cord trauma is damage to the spinal cord. It may result from direct injury to the cord itself or indirectly from disease of the surrounding bones, tissues, or blood vessels.

Injuries at any level can cause altered muscle tone (spasticity), loss of normal bowel and bladder control (may include constipation, incontinence, bladder spasms), numbness, sensory changes, pain, weakness, and paralysis. When spinal cord injuries occur in the neck area, symptoms can affect the arms, legs, and middle of the body. The symptoms may occur on one or both sides of the body. Symptoms can also include breathing difficulties from paralysis of the breathing muscles, if the injury is high up in the neck. When spinal injuries occur at chest level, symptoms can affect the legs. Injuries to the cervical or high thoracic spinal cord may also result in blood pressure problems, abnormal sweating, and trouble maintaining normal body temperature. When spinal injuries occur at the lower back level, symptoms can affect one or both legs, as well as the muscles that control the bowels and bladder.

Forms of treatment vary. Corticosteroids, such as dexamethasone or methylprednisolone, are used to reduce swelling that may damage the spinal cord. If spinal cord pressure is caused by a growth that can be removed or reduced before the spinal nerves are completely destroyed, the incidence of paralysis may be reduced. Ideally, corticosteroids should begin as soon as possible after the injury.

Surgery may be needed to remove fluid or tissue that presses on the spinal cord (decompression laminectomy); remove bone fragments, disk fragments, or foreign objects; or fuse broken spinal bones or place spinal braces.

Bedrest may be needed to allow the bones of the spine to heal.

Spinal traction may be recommended. That can help keep the spine from moving. The skull may be held in place with tongs (metal braces placed in the skull and attached to traction weights or to a harness on the body). The patient may need to wear the spine braces for a long time.

The patient may need physical therapy, occupational therapy, and other rehabilitation therapies after the injury has healed. Rehabilitation will help the patient to cope with the disability from the spinal cord injury.

Muscle spasticity can be relieved with medications taken by mouth or injected into the spinal canal. Botox injections into the muscles may also be helpful Painkillers (analgesics), muscle relaxers, and physical therapy are used to help control pain.

However, none of those treatments encourage regrowth of nerve tissue. One form of treatment that is intended to do so is magnetic nerve stimulation. Available magnetic nerve stimulation devices for stimulating sacral nerve root are indirect. Indirect magnetic stimulation of spinal segments and sacral nerve roots has been shown to promote bowel emptying of patients with spinal, cord damage as well as induce enhancement of respiratory function in tetraplegic patients. However, indirect magnetic nerve stimulation has received mixed results, possibly due to its indirectness. There are no existing devices for direct magnetic spinal nerve stimulation.

WO 2012 064968 A1 describes a motor device for bypassing or bridging an area of neurological damage, including at least one electrode having an electric mechanism for generating electric current, and a programming mechanism for programming the electrode.

WO 2008/088985 A2 describes a neuro-stimulation system.

US 2010/0324642 A1 describes a method of treating movement disorders by the modulation of neuronal transmission using time-variant non-conservative magnetic fields.

US 5,030,225 (D4) describes a medical device for use in regenerating a severed nerve. The device includes an implantable, tubular, electrically-charged membrane having openings adapted to receive the ends of the severed nerve and a lumen having a diameter ranging from about 0.5 millimeters to about 2.0 centimeters to permit regeneration of the nerve there through.

### DISCLOSURE OF THE INVENTION

It is therefore an object of the present invention which is defined in independent claim 1 to provide a medical device that surrounds a nerve and provides dual mode stimulation with both electric and magnetic stimulation modes.

To achieve the above and other objects of the present invention, described herein is a device that encircles the spinal segmental nerves like a ring and causes stimulation of the nerves. Electric stimulation of severed nerve roots has given favorable results in testing. Some testing indicates that magnetic stimulation reduces spasticity. The present invention offers magnetic as well as electric stimulation properties in order to balance the two modes. In some embodiments of the present invention, the device does not contact the nerve, and avoids nerve damage that ensues when there is direct physical contact with a nerve for extended periods of time.

Throughout this specification, the structure of the various embodiments of the present invention will be described using the terms ring and collar interchangeably. The present invention and the various embodiments described and envisioned herein use a structure that encircles the nerve, be it a ring, a collar, or similar geometry, where applicants do not wish to be bound to any specific geometric shape or size. Rather, the present invention and the various embodiments described and depicted herein provide a structure that encircles, encompasses, or otherwise surrounds a nerve or a specific portion of a nerve.

The present invention can be utilized for direct stimulation of patients with spinal trauma and stroke. The present invention and the various embodiments described and envisioned herein serve to simulate, for example, anterior roots of the spinal cord segmental nerves related to the movements. Initially, the targeted functions will be bowel emptying, bladder stimulation, and cough induction. In some embodiments of the present invention, a computer program and a stimulator will be made for all or various motor functions based on root involvement of those functions, such as limb movements.

In some embodiments of the present invention, a patient can control his or her motor movements using a remote control device. In one embodiment of the present invention, the initial mode of stimulation will be magnetic. If the outcome following magnetic stimulation is reduction of hyperreflexia or spasticity, which is a common side effect following upper motor neuron lesions like spinal cord injury or stroke, magnetic stimulation only will be insufficient to achieve full motor control. In this case, electric stimulation through electrodes along with magnetic stimulation will be applied. A balance of these two modes of stimulation will in turn control hyperreflexia and spasticity to achieve movements.

In some embodiments of the present invention, a wireless power supply and telemetric control may be provided to avoid infections and minimize the size of the device. These devices may be implanted microsurgically and may be screwed to the bone using plastic screws.

Stimulating coils may be made out of a conductive material or a conductive polymer or a plastic magnetic conductance material, for example. With the latter, a patient will be able to go through the usual MRI scans, and the like.

Immediate applications for the present invention and the various embodiments described and envisioned herein include the following:
1) Stimulate bowel movements;
2) Stimulate bladder emptying;
3) Stimulate an erection;
4) Reduce muscle spasticity following upper motor neuron lesions; and
5) Induce cough to prevent respiratory infections.

In some embodiments of the present invention, all spinal cord segments will carry rings for individual nerve stimulation, and a software program will stimulate all involved segments in an optimum stimulation pattern for motor movement, to allow for immediate motor control following peripheral nerve lesions or other maladies.

### BRIEF DESCRIPTION OF THE DRAWINGS

A preferred embodiment and variations thereof will be set forth with reference to the drawings, in which:
Figure 1 is a diagram showing the placement of one embodiment of the stimulation collar in a patient's nervous system;
Figure 2 is a diagram showing placement of the stimulation collar:
Figure 3A is a close-up view of the stimulation collar on a nerve:
Figure 3B is a cross-sectional view of the stimulation collar taken along line A-A of Figure 3A; and
Figure 4 is a diagram showing an alternative placement of the stimulation collar.

### BEST MODE FOR CARRYING OUT THE INVENTION

A preferred embodiment of the present invention and variations thereof will be set forth in detail with reference to the drawings, in which like reference numerals refer to like elements or steps throughout.

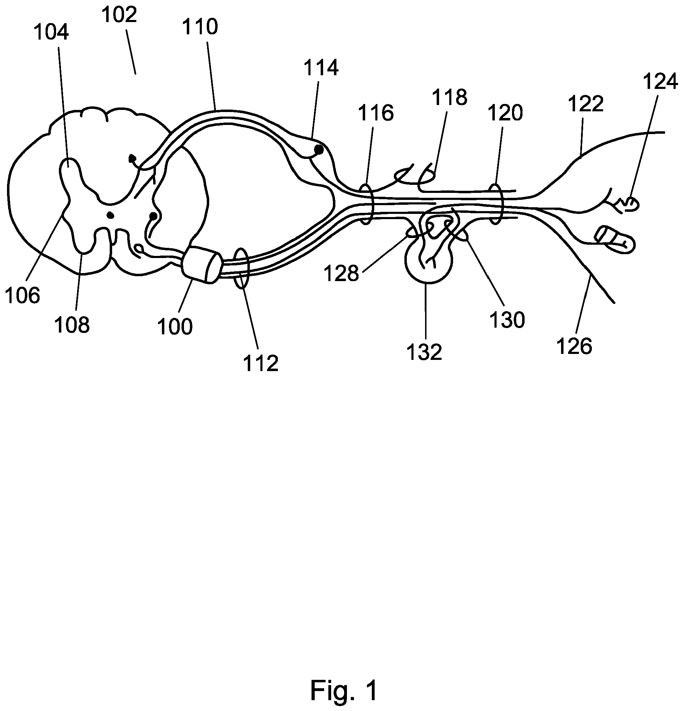

Figure 1 shows an example of placement of a spinal stimulation collar or ring 100. The spinal cord 102 (shown here in cross section) includes a dorsal (posterior) horn 104 of spinal gray matter; a lateral horn 106, found only in segments T1-L2, of preganglionic sympathetic neurons; and a ventral (anterior) horn 108 containing motor neurons. Dorsal and ventral roots 110, 112 extend from the dorsal and ventral horns 104, 108 respectively. The dorsal root includes a dorsal ganglion 114. The roots 110, 112 join to form a spinal nerve 116. which branches into a dorsal primary ramus 118, which goes to the skin and muscles of the back, and a ventral primary ramus 120, which splits into sensory fibers 122, postganglionic sympathetic innervation (into glands and blood vessels) 124. and motor axons 126 to skeletal muscle. The ventral primary ramus 120 is also connected via a gray ramus communicans 128 and a white ramus communicans 130 to a sympathetic (paravertebral) ganglion 132.

The stimulation collars 100 surround the ventral nerve roots 112 and are, in some embodiment of the present invention, attached to the bone by microscrews or other suitable techniques. The spinal stimulation collar 100 provides for electric and magnetic stimulation of the nerves. A stimulator is used for applying stimulation to the stimulation collar, and comprises a combination of both electric and magnetic stimulation. The stimulator may comprise a coil, an electrode, or a material that provides the necessary electric or magnetic stimulation. The spinal stimulation collar 100 may comprise an electrically conductive material such as a stainless steel or nitinol that is electrically connected to an electric field source such as a battery, ultracapacitor, or the like. In some embodiments of the present invention, the electric field is a constant field. In other embodiments, the electric field may be pulsed, sinusoidal, sporadic, or otherwise temporally patterned or intermittent. The electric field may also, in some embodiments of the present invention, change polarity, gradient, strength, polarization, direction, or other parameters.

In some embodiments of the present invention, the spinal stimulation collar may generate an electric field independent of an external electromotive source. For example, the spinal stimulation collar 100 may include an electret material such as properly processed quartz, silicon dioxides, synthetic polymers such as fluoropolymers, polypropylene, polyethyleneterephthalate, polytetrafluoroethylene, parylene, and the like. An exemplary process for making electrets is disclosed in United States Patent 4,291,245 to Nowlin et al. and entitled "Elecrets". The spinal stimulation collar 100 may also comprise an electric field generating material such as a piezoelectric material that generates an electric field upon the application of an external force, such as body movements. Suitable piezoelectric materials include, for example, barium titanate, lead titanate, gallium orthophosphate, bismuth ferrite, sodium potassium niobate, sodium niobate, polyvinylidene fluoride, aluminum nitride, or the like. Electroactive polymers may also be used to provide an electric field generating coating or structure for the stimulation collar, and may be applied in conjunction with another material such as a piezoelectric material, electret material, or the like. Electroactive polymers may be dielectric or ionic, and may, in one embodiment of the present invention, be a ferroelectric polymer where they maintain a permanent electric polarization that may be reversed in the presence of an external electric field. One example of a ferroelectric polymer is polyvinylidene fluoride. Dielectric electroactive polymers include, for example, silicones and acrylic elastomers such as the acrylic elastomer VHB4910 available from the 3M Company, St. Paul, Minnesota. Examples of ionic electroactive polymers include conductive polymers and ionic polymer-metal composites. Polyelectrolytes may also be used as an electric field generating material in accordance with the present invention. Polyelectrolytes are polymers whose repeating units have an electrolyte group. The polymers become charged when dissociated in water. For example, polyacrylic acid is negatively charged when dissociated in water, as is poly(sodium styrene sulfonate). Galvani potential materials may further be used to provide an electric field generating coating or structure for a medical device in accordance with the present invention. Dissimilar metals may be joined together as a layer or composite structure. When two different metals make electrical contact, electrons flow from the metal with the lower work function to the metal with the higher work function, and an electric double layer is formed at the interface. In addition, semiconductor materials may be used and may, in some embodiments of the present invention. be combined with other materials such as electret materials, conductive materials, piezoelectric materials, and the like

In addition to an electric field generating material or an electric field source and associated electrode material the stimulation collar contains a magnetic field component. The magnetic field source may be, in some embodiments of the present invention, a coil to provide a source of a magnetic field. Other embodiments of the present invention may use permanent magnet materials such as, for example, ferrite, alnico, rare earth materials, and the like. The stimulation collar may also contain a battery, capacitor, or other electrical charge storage device that is electrically connected to the coil. The stimulation collar may, in one embodiment of the present invention, continuously generate a low intensity magnetic field (0.01 Tesla, for example). The coil may, in one embodiment of the present invention, be a Helmholtz coil. In other embodiments of the present invention, the magnetic field may be pulsed, sinusoidal, sporadic, or otherwise temporally patterned or intermittent. The magnetic field may also, in some embodiments of the present invention, change polarity, gradient, strength, polarization, direction, or other parameters.

Both the magnetic and electric field components of the present invention may be under the control of a microprocessor based controller to turn the field on and off, change the magnitude, duration, frequency, or other attributes of the field. In some embodiments of the present invention, there is only an electric field. In other embodiments of the present invention, there is only a magnetic field.

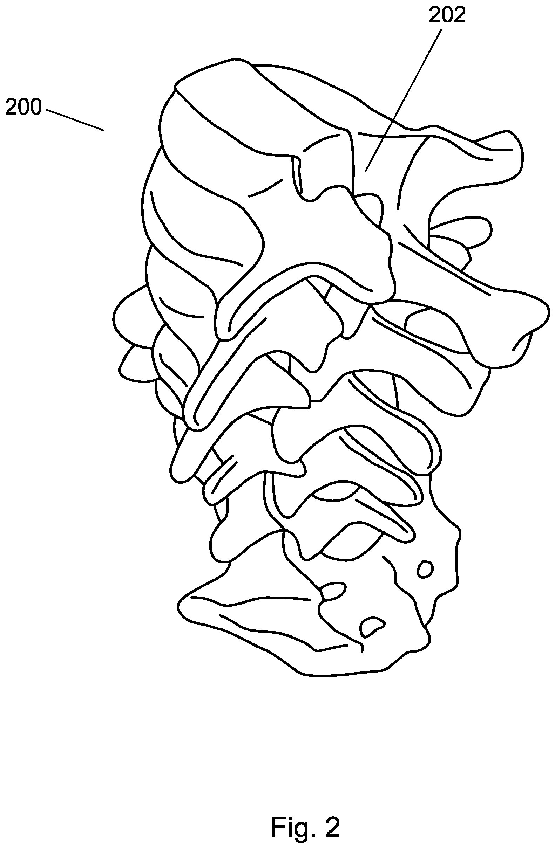

Figure 2 shows the spine 200 defining the spinal canal 202. The posteriors of the vertebral bodies and the vertebral arches form the bony border of the spinal canal 202. The spinal canal 202 contains the spinal cord 102 and the nerve roots 110, 112. A spinal stimulating collar 100 is attached as shown in Figure 1.

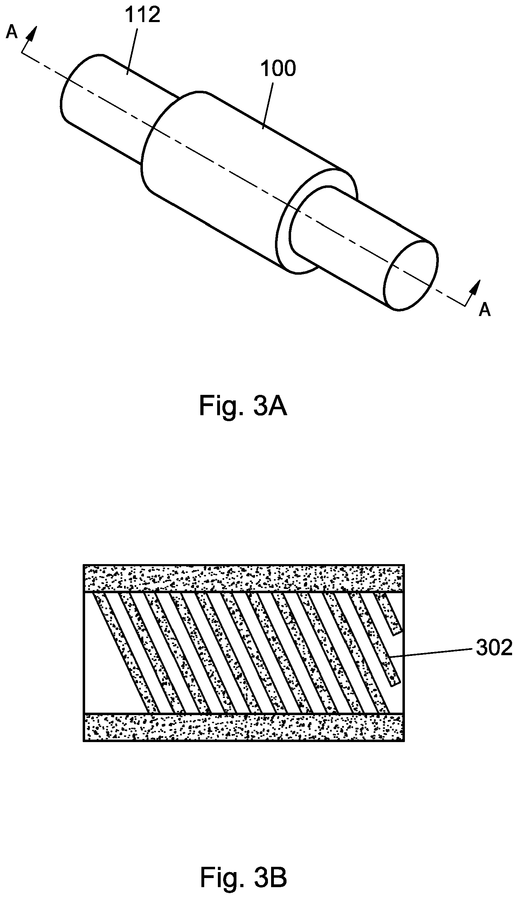

Figure 3A shows a collar 100 surrounding a ventral root 112. Figure 3B shows a coronal section of the collar 100. including stimulating magnetic fibers 302. The stimulating magnetic fibers 302 may, in some embodiments of the present invention, be generally parallel to each other within or upon a biocompatible material. In other embodiments of the present invention, the magnetic fibers 302 may be random or pseudo-random in distribution within or upon a biocompatible material. The stimulation collar 100 may, in some embodiments of the present invention, be cylindrical. The stimulation collar 100 may also contain an opening, break, or other structural discontinuity to facilitate surgical placement around the nerve root. The stimulation collar 100 may then be closed around the nerve by way of sutures, pins, screws, hinges, rivets, flanges, bolts, flares, interlocking elements, push fit joints, or the like.

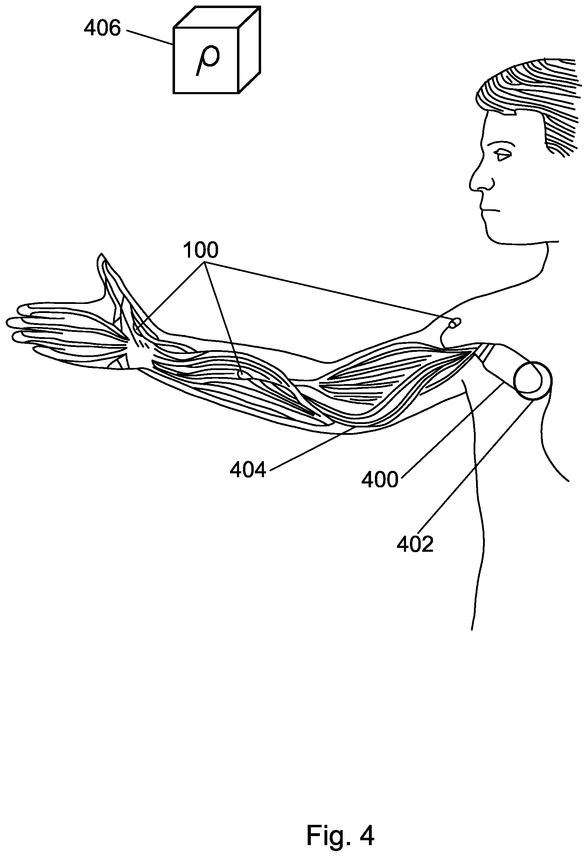

Figure 4 shows the use of one preferred embodiment of the present invention to surround nerve roots during peripheral nerve repair. An implanted stimulator 400 includes a transmitting coil 402 for control and is connected via electrode leads 404 to collars or rings 100. The coil 402 allows wireless communication with a processor 406 for control. Such a stimulator 400 and processor 406 can be used in many embodiments of the present invention to provide the correct stimulation needed for therapeutic effect.

It is, therefore, apparent that there has been provided, in accordance with the various objects of the present invention, spinal nerve stimulation rings for rehabilitation of patients with spinal trauma and stroke.

## Claims

1. A device for applying treatment to a nerve (112, 116) of a patient, the device comprising:
a stimulating collar (100) configured for being implanted around the nerve (112, 116); and
a stimulator (400) for applying electrical as well as magnetic stimulation to the nerve (112, 116) through the collar (100).

2. The device of claim 1, wherein the stimulator (400) is configured to first apply magnetic stimulation, receive an outcome from the magnetic stimulation, and then apply electrical stimulation based on the received outcome.

3. The device of claim 2, wherein the received outcome is a reduction in patient hyperreflexia.

4. The device of claim 2, wherein the received outcome is a reduction in patient spasticity.

5. The device of claim 1, wherein the stimulator (400) comprises a communication coil (402), and wherein the stimulator (400) is configured to operate under telemetric control.

6. The device of claim 1, wherein the stimulating collar (100) comprises a stimulating coil made out of a plastic magnetic conductance material.

7. The device of claim 1, wherein the stimulating collar (100) comprises stimulating magnetic fibers (302).

## Patentansprüche

1. Vorrichtung zum Anwenden einer Behandlung auf einen Nerv (112, 116) eines Patienten, wobei die Vorrichtung aufweist:
eine Stimulationsmanschette (100), die dafür konfiguriert ist, um den Nerv (112, 116) herum implantiert zu werden; und
einen Stimulator (400) zum Anwenden einer elektrischen sowie einer magnetischen Stimulation auf den Nerv (112, 116) durch die Manschette (100).

2. Vorrichtung nach Anspruch 1, wobei der Stimulator (400) dafür konfiguriert ist, zuerst eine magnetische Stimulation anzuwenden, ein Ergebnis von der magnetischen Stimulation zu empfangen und dann basierend auf dem empfangenen Ergebnis eine elektrische Stimulation anzuwenden.

3. Vorrichtung nach Anspruch 2, wobei das empfangene Ergebnis eine Verminderung der Hyperreflexie des Patienten ist.

4. Vorrichtung nach Anspruch 2, wobei das empfangene Ergebnis eine Verminderung der Spastizität des Patienten ist.

5. Vorrichtung nach Anspruch 1, wobei der Stimulator (400) eine Kommunikationsspule (402) aufweist, und wobei der Stimulator (400) dafür konfiguriert ist, unter einer telemetrischen Steuerung zu arbeiten.

6. Vorrichtung nach Anspruch 1, wobei die Stimulationsmanschette (100) eine Stimulationsspule aufweist, die aus einem magnetisch leitfähigen Kunststoffmaterial hergestellt ist.

7. Vorrichtung nach Anspruch 1, wobei die Stimulationsmanschette (100) stimulierende magnetische Fasern (302) aufweist.

## Revendications

1. Dispositif pour appliquer un traitement à un nerf (112, 116) d'un patient, le dispositif comprenant :
un collier stimulant (100) configuré pour être implanté autour du nerf (112, 116) ; et
un stimulateur (400) pour appliquer une stimulation électrique et magnétique au nerf (112, 116) à travers le collier (100).

2. Dispositif selon la revendication 1, dans lequel le stimulateur (400) est configuré pour appliquer en premier une stimulation magnétique, et puis appliquer une stimulation électrique basée sur le résultat reçu.

3. Dispositif selon la revendication 2, dans lequel le résultat reçu est une réduction d'une hyper réflectivité du patient.

4. Dispositif selon la revendication 2, dans lequel le résultat reçu est une réduction d'une spasticité du patient.

5. Dispositif selon la revendication 1, dans lequel le stimulateur (400) comprend une bobine de communication (402), et dans lequel le stimulateur (400) est configuré pour fonctionner sous contrôle télémétrique.

6. Dispositif selon la revendication 1, dans lequel le collier stimulant (100) comprend une bobine de stimulation faite d'un matériau plastique à conductance magnétique.

7. Dispositif selon la revendication 1, dans lequel le collier stimulant (100) comprend des fibres magnétiques stimulantes (302).
